(19) Europäisches Patentamt
European Patent Office
Office européen des brevets

(11) EP 2 586 422 A1

(12) EUROPEAN PATENT APPLICATION
published in accordance with Art. 153(4) EPC

(43) Date of publication:
**01.05.2013 Bulletin 2013/18**

(21) Application number: **11798251.2**

(22) Date of filing: **24.06.2011**

(51) Int Cl.:
***A61K 8/60*** *(2006.01)* ***A61K 8/20*** *(2006.01)*
***A61K 8/34*** *(2006.01)* ***A61K 8/81*** *(2006.01)*
***A61K 8/86*** *(2006.01)* ***A61K 8/87*** *(2006.01)*
***A61Q 5/06*** *(2006.01)*

(86) International application number:
**PCT/JP2011/064519**

(87) International publication number:
**WO 2011/162370 (29.12.2011 Gazette 2011/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.06.2011 JP 2011139940**
**25.06.2010 JP 2010144376**

(71) Applicant: **Shiseido Company, Ltd.**
**Chuo-ku**
**Tokyo 104-8010 (JP)**

(72) Inventors:
- **KURASHIMA, Takumi**
  **Yokohama-shi**
  **Kanagawa 224-8558 (JP)**
- **FUJIYAMA, Taizo**
  **Yokohama-shi**
  **Kanagawa 224-8558 (JP)**

(74) Representative: **Takeuchi, Maya et al**
**S.A. Fédit-Loriot**
**38, avenue Hoche**
**75008 Paris (FR)**

# (54) HAIR STYLING COSMETIC

(57) Problem

To provide a hair styling cosmetic composition which is excellent in hair styling property and hair restyling property, even though having a low viscosity, and is excellent in non-stickiness, smoothness, and light finish of the hair.

Means for Resolution

A hair styling cosmetic composition comprising (a) one or more substances being solid at room temperature (25°C) selected from among ($a_1$) a sugar alcohol, ($a_2$) a sugar, ($a_3$) a polyalkylene glycol polymer, and ($a_4$) an inorganic salt, in an amount of from 0.1 to 20% by mass, (b) a sugar derivative and/or a sugar alcohol derivative being liquid at room temperature in an amount of from 0.1 to 25% by mass, (c) one or more film-forming polymers selected from among acrylic and vinylic film-forming polymers (but excepting ampholytic polymers) and urethanic polymers, in an amount of from 0.1 to 12% by mass, and (d) an aqueous solvent, wherein the viscosity of the system is at most 10,000 mPa·s (at 25°C with B-type viscometer).

EP 2 586 422 A1

**Description**

TECHNICAL FIELD

The present invention relates to a hair styling cosmetic composition. More precisely, the invention relates to a hair styling cosmetic composition which is excellent in hair styling property and hair restyling property, even though having a low viscosity, and is excellent in non-stickiness, smoothness, and light finish of the hair.

BACKGROUND ART

Heretofore, in hair styling cosmetic compositions, hair styling resins such as hair-fixing polymer, a film-forming polymer or the like are incorporated for styling. However, hair styling resins are problematic in that they cause stiffness, non-uniformity of films applied on the hair, and reduction in the styling retentivity in high-humidity environments. Accordingly, for solving those problems, various countermeasures have been taken.

For example, JP 2004-505902A (Patent Reference 1) describes that hair care composition containing a specific water-soluble polyalkylene glycol and a film-forming polymer in a specific ratio and further containing a liquid carrier is excellent in a hair restyling property and gives an improved feeling. However, the hair care composition in the Patent Reference 1 is problematic in that it could not obtain sufficient hair styling property (easiness in hair styling).

JP 2007-217314A (Patent Reference 2) describes that a misty powder cosmetic composition containing a polymer compound for hair fixation, a polyalcohol, a monoalcohol and a propellant each in a specific amount is excellent in hair restyling property without stickiness and has a natural glossy appearance. However, the misty powder cosmetic composition in the Patent Reference 2 is problematic in that it could not obtain sufficient hair styling properties (easiness in hair styling), and was not easily washed off from the hair.

JP 3-261713A (Patent Reference 3) describes that a hair cosmetic containing a specific polyoxyalkylene compound and/or polyoxyalkylene alkyl glucoside, a polymer compound for hair fixation, and a high-molecular-weight polyethylene glycol (molecular weight, 6,000 to 30, 000) has a hair styling property and give smoothness or evenness. However, the hair cosmetic in the Patent Reference 3 is problematic in that it could not obtain sufficient hair styling property (easiness in hair styling).

JP 2002-167317A (Patent Reference 4) describes that a hair cosmetic composition containing an ampholytic polymer, a sugar alcohol, and a sugar alcohol derivative (e.g., polyoxyalkylene adduct to sugar alcohol) has a hair styling property and a hair set retention property, and is free from sticky-feeling and stiff-feeling. However, the hair cosmetic composition in the Patent Reference 4 is problematic in that it could not obtain sufficient hair arranging property, and was not easily washed off from the hair.

It is especially difficult for a water-based low-viscosity hair styling cosmetic composition to have both a hair styling property (easiness in hair styling) and a hair restyling property. Accordingly, it has been desired to develop a water-based low-viscosity hair styling cosmetic composition that has both hair styling property and hair restyling property, as well as has a good feeling in use.

PRIOR ART REFERENCES

PATENT REFERENCES

Patent Reference 1: JP 2004-505902A
Patent Reference 2: JP 2007-217314A
Patent Reference 3: JP 3-261713A
Patent Reference 4: JP 2002-167317A

SUMMARY OF INVENTION

PROBLEMS THAT INVENTION IS TO SOLVE

The present invention has been made in consideration of the above-mentioned situation, and its object is to provide a hair styling cosmetic composition which is excellent in hair styling performance and hair restyling performance, even though having a low viscosity, and is excellent in non-stickiness, smoothness, and light finish of the hair.

MEANS FOR SOLVING THE PROBLEMS

In order to solve the above-mentioned problems, the invention provides a hair styling cosmetic composition comprising (a) one or more substances being solid at room temperature (25°C) selected from among ($a_1$) a sugar alcohol, ($a_2$) a sugar, ($a_3$) a polyalkylene glycol polymer, and ($a_4$) an inorganic salt, in an amount of from 0.1 to 20% by mass, (b) a sugar derivative and/or a sugar alcohol derivative being liquid at room temperature in an amount of from 0.1 to 25% by mass, (c) one or more film-forming polymers selected from among acrylic and vinylic film-forming polymers (but excepting ampholytic polymers) and urethanic polymers, in an amount of from 0.1 to 12% by mass, and (d) an aqueous solvent, wherein the viscosity of the system is at most 10,000 mPa·s (at 25°C with B-type viscometer).

ADVANTAGEOUS EFFECTS OF INVENTION

According to the invention, there is provided a hair styling cosmetic composition which is excellent in hair styling property and hair set retention property, even though having a low viscosity, and is excellent in non-stickiness, smoothness, and lightly-finishing of the hair.

MODE FOR CARRYING OUT THE INVENTION

The invention is described in detail hereinunder. In the following, POE means polyoxyethylene, POP means polyoxypropylene, and POB means polyoxybutylene.

[Component (a)]

Component (a) is one or more substances that are solid at room temperature(25°C) and selected from among ($a_1$) a sugar alcohol, ($a_2$) a sugar, ($a_3$) a polyalkylene glycol polymer, and ($a_4$) an inorganic salt.

<Component ($a_1$) >

The sugar alcohol solid at room temperature is a polyalcohol which is in a solid state at room temperature to be obtained through reduction of the carbonyl group of sugar. Concretely, exemplary sugar alcohol includes maltitol ("Malbit"; by B Food Science Co., Ltd.), sorbitol ("Sorbitol C"; by B Food Science Co., Ltd.), ribitol, mannitol, arabitol, galactitol, xylitol, erythritol, and inositol. Above all, preferred are sorbitol and maltitol from the viewpoint of non-stickiness and non-stiffness.

<Component ($a_2$) >

The sugar solid at room temperature may be, not specifically defined, any one capable of being generally incorporated in cosmetic compositions, and includes monoses (e.g., aldose, and ketose, etc.), trioses (trisaccharides; e.g., glyceryl aldehyde, and dihydroxyacetone, etc.), tetroses (tetrasaccharides; e.g., erythrose, threose, and erythrulose, etc.), pentoses (pentasaccharides; e.g., ribose, lyxose, xylose, arabinose, apiose, ribulose, and xylulose, etc.), hexoses (hexasaccharides; e.g., glucose (=grape sugar), mannose, galactose, idose, fructose (= fruit sugar), and sorbose) , and heptoses (heptasaccharides; e.g., sedoheptulose, and coriose, etc.). Above all, preferred is fructose (= fruit sugar) as is non-stickiness and is excellent in hair styling property.

<Component ($a_3$) >

Preferred examples of the polyalkylene glycol polymer that is solid at room temperature include an EO polymer composed of ethylene oxide (EO) constitutive units polymerized, a PO polymer composed of propylene oxide (PO) constitutive units polymerized, a BO polymer composed of butylene oxide (BO) constitutive units polymerized, as well as the corresponding copolymers composed of the respective constitutive units copolymerized. Especially preferred ones include an EO polymer, an EO/PO copolymer containing EO constitutive units and PO constitutive units, and an EO/BO copolymer containing EO constitutive units and BO constitutive units. The type of copolymerization is not specifically defined, including any of block copolymerization, graft copolymerization, random copolymerization, etc.

As the EO polymer, preferred is polyethylene glycol (PEG) having a mass-average molecular weight (Mw; hereinafter this may be simply referred to as "molecular weight") of at least 1,000. Not specifically defined, the uppermost limit of the molecular weight is preferably up to about 20,000 or so. In concrete terms, exemplary ones include PEG having a molecular weight of 1,000 (hereinafter this may be expressed as "PEG 1,000"), PEG 1,540, PEG 2, 000, PEG 4, 000, PEG 6, 000, PEG 8, 000, PEG 10,000, PEG 11,000, and PEG 20,000 . Above all, PEG 1,000 to 10, 000 are

more preferred, PEG 1, 000 to 8, 000 are even more preferred, and PEG 1,000 to 6,000 are especially preferred from the viewpoint of the hair styling property and hair restyling property.

Preferred examples of the EO/PO copolymer include EO/PO block copolymers represented by the following formula (XII):

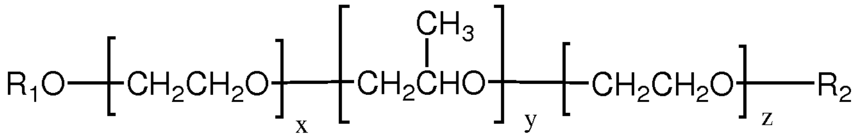

(XII)

In the above formula (XII), the substituents and the symbols have the following meanings.

$R_1$ and $R_2$ each independently represent an alkyl group having from 1 to 4 carbon atoms, or a hydrogen atom. The alkyl group having from 1 to 4 carbon atoms includes a methyl group, an ethyl group, an n-propyl group, an isopropyl group, an n-butyl group, a sec-butyl group, and a tert-butyl group. Preferred are a methyl group and an ethyl group. An alkyl group having 5 or more carbon atoms may lower the hydrophilicity of the copolymer and tend to lower the moisturizing feeling.

(x + z) means the added molar number of the EO constitutive units; and y means the added molar number of the PO constitutive units. [(x +z)/(x+z+y)] is from 0.2 to 0.8, preferably from 0.4 to 0. 7. When the proportion of the EO constitutive units to the (EO + PO) constitutive units is less than 0.2, then the smoothness tends to be poor; but when more than 0.8, then the stickiness tends to occur.

$60 \leq x + y + z \leq 100$. When (x + y + z) is less than 60, then some types of component ($a_3$) may be liquid; but when more than 100, then the composition may be sticky and the solubility thereof may be poor.

Specific examples of the EO/PO block copolymer represented by the above formula (XII) include POE(35)/POP (40) block copolymer dimethyl ether, and POE(50)/POP(40) block copolymer dimethyl ether.

Preferred examples of the EO/BO copolymer include EO/BO block copolymers represented by the following formula (XIII):

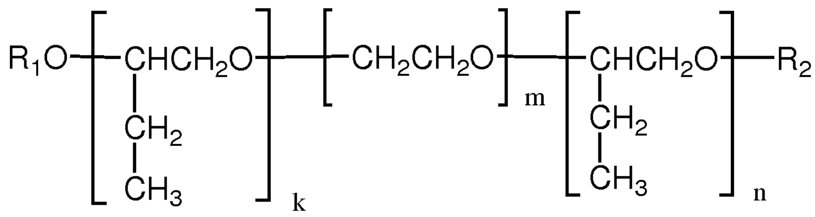

(XIII)

In the formula (XIII), $R_1$ and $R_2$ have the same meanings as in the above formula (XII).

m means the added molar number of the EO constitutive units; and (k + n) means the added molar number of the BO constitutive units. [m/(m + k +n)] is from 0.2 to 0.9, preferably from 0.4 to 0.9. When the proportion of the EO constitutive units to the (EO + BO) constitutive units is less than 0.2, then the smoothness tends to be poor; but when more than 0.9, then the sticky-feeling tends to occur.

Preferably, $60 \leq m + k + n \leq 100$ or so. When (m + k + n) is less than 60, then some types of component ($a_3$) may be liquid; but when more than 100, then the sticky-feeling tends to occur and the solubility may be poor.

Specific examples of the EO/BO block copolymer represented by the above formula (XIII) include POE(52) /POB(32) block copolymer dimethyl ether, and POE(73)/POB(11) block copolymer dimethyl ether.

<Component ($a_4$) >

The salt that is solid at room temperature may be, not specifically defined, any one capable of being generally incorporated in cosmetic compositions, and alkali metal salts, alkaline earth metal salts and the like are preferred for use herein. Preferred examples include magnesium chloride, sodium chloride, and manganese chloride.

In the hair styling cosmetic composition of the invention, component (a) mainly contributes to the hair setting property, the hair arranging property, light finish of the hair, and the hair-pliability property. Component (a) is solid at room temperature, and therefore, after the application of the hair styling cosmetic composition on the hair, followed by being volatilized away the solvent from the applied composition, the solid component (a) can remain as widely covering on the hair, and efficacy of component (a) sustain effectively. As described below, the hair styling cosmetic composition

of the invention is water-based and has a low viscosity, and hence, the ingredients therein can be widely and evenly applied on the hair.

As component (a), preferred is use of component ($a_1$) , component ($a_3$), component ($a_2$) and component ($a_4$) in that order. In the invention, preferred is an embodiment where component (a) contains at least component ($a_1$) and/or component ($a_3$).

The amount of component (a) to be used in the hair styling cosmetic composition of the invention is from 0.1 to 20% by mass, preferably from 3 to 8% by mass. When the amount is less than 0.1% by mass, then component (a) could not sufficiently exert its property; but on the other hand, even though the amount is more than 20% by mass, not only there is no expecting a further improvement in efficacy that is commensurate with the increased amount of its use, but also the viscosity of the composition may increase, which is therefore unfavorable in point of the oily feeling, stickiness, and heavy finish of the hair.

[Component (b)]

Component (b) is a sugar derivative and/or a sugar alcohol derivative that are liquid at room temperature. Exemplary sugar derivative include glucose derivatives, etc.; and exemplary sugar alcohol derivative include pentahydric or hexahydric sugar alcohol derivatives (e.g., sorbitol derivatives, mannitol derivatives, and xylitol derivatives, etc.), but not limited thereto. In particular, in the invention, sugar or sugar alcohol alkylene oxide addition polymers are preferred. As the alkylene oxide for addition polymerization, preferably used here are constituent units of EO, PO, and BO, etc.

The "sugar or sugar alcohol alkylene oxide addition polymer that is liquid at room temperature" concretely includes POP sorbitol (as commercial products, "Uniol HS-1600D" (by NOF Corp.), etc.), POE (10) methylglucoside (as commercial products, "Glucam E-10" (by Lubrizol Japan Limited), etc.), and POP(20) methylglucoside (as commercial products, "Glucam P-20" (by Lubrizol Japan Limited), etc.).

The amount of component (b) to be used in the hair styling cosmetic composition of the invention is from 0.1 to 25% by mass, preferably from 3 to 15% by mass. When the amount is less than 0.1% by mass, then component (b) could not sufficiently exert its property; but on the other hand, even though the amount is more than 25% by mass, not only there is no expecting a further improvement in efficacy that is commensurate with the increased amount of its use, but also the viscosity of the composition may increase, which is therefore unfavorable in point of stickiness, and heavy finish of the hair.

[Component (c)]

As component (c), in the invention, used are one or more selected from acrylic and vinylic film-forming polymers (but excepting ampholytic polymers) and urethanic polymers among film-forming polymers that have heretofore been used for hair styling cosmetic composition such as hair styling agents and others, especially from the viewpoint of the hair restyling property thereof.

<Acrylic or Vinylic Film-Forming Polymer>

Any one among anionic, cationic, and nonionic polymers may be used.

Exemplary anionic polymers include alkyl acrylate/diacetone acrylamide copolymer [Plas Cize L-53P, Plas Cize L-9909B, Plas Cize L-9948B, etc. (all by Goo Chemical Co., Ltd.)], alkyl acrylate/octylacrylamide copolymer [Dermacryl 79 (by AkzoNobel)], polyethylene glycol/polypropylene glycol-25/dimethicone acrylate copolymer [Rubiflex SILK (by BASF)], acrylic acid/acrylic acid amide/ethyl acrylate copolymer [Ultrahold 8, Ultrahold Strong (both by BASF)], and alkyl acrylate copolymer [Aniset NF-1000, Aniset HS-3000, etc. (all by Osaka Organic Chemical Industry Ltd.)].

Exemplary cationic polymers include vinylpyrrolidone/dimethylaminoethyl methacrylate copolymer diethyl sulfate salt [H.C. Polymer 1S(M), H.C. Polymer 2 (both by Osaka Organic Chemical Industry Ltd.), Gafquat 755N (by ISP)], vinylpyrrolidone/dimethylaminopropylmethacrylamide/lauryldimethylaminopropylm ethacrylamide copolymer [Styleze W-20 (by ISP)], vinylpyrrolidone/N,N-dimethylaminoethyl methacrylate/alkyl acrylate/tripropylene glycol diacrylate copolymer [Cosquat GA467, Cosquat GA468 (both by Osaka Organic Chemical Industry Ltd.)], polydime-thylmethylenepiperidinium chloride [Merquat 100 (by Nalco)], dimethyldiallylammonium chloride/acrylamide copolymer [Merquat 550 (by Nalco)], and trimethylaminopropylacrylamide chloride/dimethylacrylamide copolymer.

Exemplary nonionic polymers include polyvinylpyrrolidone [Luviskol K17, Luviskol K30, Luviskol K90 (all by BASF), PVP K (by ISP)], vinylpyrrolidone/vinyl acetate copolymer [PVP/VA S-630, PVP/VA E-735, PVP/VA E-335 (all by ISP), Luviskol VA73W, Luviskol 37E (both by BASF), PVA-6450 (by Osaka Organic Chemical Industry Ltd.)], vinyl methyl ether/alkyl maleate copolymer [Gauntlets A-425, Gauntlets ES-225, Gauntlets ES-335 (all by ISP)], and vinylpyrrolidone/methacrylamide/vinylimidazole copolymer [Luviset Clear (by BASF)].

In the invention, mainly from the viewpoint of the hair restyling property, ampholytic polymers are not used as

the acrylic and vinylic film-forming polymers. Those ampholytic polymers include acrylic acid octylamide/hydroxypropylpropyl acrylate/butylaminoethyl methacrylate copolymer, methacryloyloxyethylcarboxybetaine/alkyl methacrylate copolymer, dimethyldiallylammonium chloride/acrylic acid copolymer, and dimethyldiallylammonium chloride/acrylamide/acrylic acid copolymer.

<Urethanic Film-Forming Polymer>

Exemplary urethanic film-forming polymers include silicone/polyether polyurethane resin [Yodosol PUD, by AkzoNobel], "Luviset P.U.R." (by BASF), and silylated urethanic polymer described in JP 2006-213706A. Exemplary acrylic-urethanic film forming polymers include "DynamX" (by AkzoNovel).

In the hair styling cosmetic composition of the present invention, component (c) mainly contributes to the hair setting property, the hair restyling property, and light finish of the hair. Component (c) may be used either alone or in combination.

The amount of component (c) to be used in the hair styling cosmetic composition of the invention is from 0.1 to 12% by mass, preferably from 1 to 6% by mass. When the amount is less than 0.1% by mass, then component (c) could not sufficiently exert its property; but on the other hand, even though the amount is more than 12% by mass, not only there is no expecting a further improvement in efficacy that is commensurate with the increased amount of its use, but also the viscosity of the composition may increase, which is therefore unfavorable in point of stiffness of the hair.

In addition to the above-mentioned component (c), the hair styling cosmetic composition of the invention may further contain polysaccharide-type polymers. Exemplary polysaccharide-type polymers include gum arabic, glucan, succinoglycan, carrageenan, karaya gum, tragacanth gum, guar gum, locust bean gum, galactomannan gum, xanthane gum, starch, carob gum, quince seed (Cydonia oblonga) , casein, dextrin, gelatin, sodium pectate, sodium alginate, methyl cellulose, ethyl cellulose, carboxymethyl cellulose, hydroxyethyl cellulose, hydroxypropyl cellulose, crystalline cellulose, O-[2-hydroxy-3-(trimethylammonio)propyl]hydroxyethyl cellulose chloride, O-[2-hydroxy-3-(trimethylammonio)propyl]guar gum chloride, O-[2-hydroxy-3-(trimethylammonio)propyl]locust bean gum chloride, and hydroxypropyltrimethylammonium chloride starch.

In the case where the polysaccharide-type polymer is incorporated, its amount to be added may be suitably controlled in consideration of the viscosity control, the hair styling property, and light finish of the hair. Preferably, the polymer amount is from 0.01 to 1% by mass, more preferably from 0.05 to 0.5% by mass in the hair styling cosmetic composition.

The hair styling cosmetic composition of the invention may further contain a surfactant that is solid (solid form) at room temperature (25°C) , if desired, from the view of hair styling property, hair restyling property, etc.

The surfactant that is solid (solid form) at room temperature (25°C) includes anionic surfactants, cationic surfactants, ampholytic surfactants, and nonionic surfactants. Nonionic surfactants are preferred for use in the invention. Specific examples of the nonionic surfactants used in the invention suitably include the ones that are solid at room temperature selected from among the nonionic surfactants exemplified below:

1. Polyoxyethylene monoalkyl ether represented by the following formula (I):

$$RO\text{-}(C_2H_4O)_n\text{-}H \qquad (I)$$

[In the formula (I), R represents an alkyl group having from 4 to 24 carbon atoms; n means the added molar number of ethylene oxide, indicating a number of from 2 to 100.]
Concretely, exemplary ones include POE lauryl ether (as commercial products, "Nonion K-220", etc.), POE cetyl ether (as commercial products, "Nonion P-210", etc.), POE oleyl ether (as commercial products, "Nonion E-215", etc.), POE stearyl ether (as commercial products, "Nonion S-215", etc.), and POE tridecyl ether (as commercial products, "Nonion T-208", etc.) (the above are all by NOF Corp.).

2. Polyoxypropylene alkyl ether represented by the following formula (II) :

$$RO\text{-}(C_3H_6O)_n\text{-}H \qquad (II)$$

[In the formula (II), R represents an alkyl group having from 4 to 24 carbon atoms; n means the added molar number of propylene oxide, indicating a number of from 2 to 100.]

Concretely, exemplary ones include POP alkyl ether (as commercial products, "Unilube MB-7", etc.), and POP stearyl ether (as commercial products, "Unilube MS-70K", etc.) (all by NOF Corp.).

3. Polyoxyethylene polyoxypropylene alkyl ether represented by the following formula (III):

$$RO\text{-}(C_2H_4O)_m(C_3H_6O)_n\text{-}H \qquad (III)$$

[In the formula (III), R represents an alkyl group having from 4 to 24 carbon atoms; m means the added molar number of ethylene oxide, indicating a number of from 2 to 100; n means the added molar number of propylene oxide, indicating a number of from 2 to 100.]
Concretely, exemplary ones include POE/POP stearyl ether (as commercial products, "Unisafe 34S-23" (by NOF Corp.), etc.), and POE/POP phytosterol (as commercial products, "Nikkol BPS-3007") (by Nikko Chemicals Co., Ltd.), etc.).

4. Polyoxyethylene monoester represented by the following formula (IV) :

$$RCOO\text{-}(\,C_2H_4O)_n\text{-}H \qquad (IV)$$

[In the formula (IV), R represents an alkyl group having from 4 to 24 carbon atoms; n means the added molar number of ethylene oxide, indicating a number of from 2 to 100.]
Concretely, exemplary ones include polyethylene glycol monooleate (as commercial products, "Nonion 0-4") (by NOF Corp.).

5. Polypropylene glycol monoester represented by the following formula (V):

$$RCOO\text{-}(\,C_3H_6O)_n\text{-}H \qquad (V)$$

[In the formula (V), R represents an alkyl group having from 4 to 24 carbon atoms; n means the added molar number of propylene oxide, indicating a number of from 2 to 100.]
Concretely, exemplary ones include polyethylene glycol monostearate (as commercial products, "BLAUNON S-400A") (by Aoki Oil Industrial Co. Ltd.).

6. Polybutylene glycol monoester represented by the following formula (VI) :

$$RCOO\text{-}(C4H_8O)_n\text{-}H \qquad (VI)$$

[In the formula (VI), R represents an alkyl group having from 4 to 24 carbon atoms; n means the added molar number of butylene oxide, indicating a number of from 1 to 100.]
Concretely, exemplary ones include butylene glycol stearate (as commercial products, "Comupoal BS") (by NOF Corp.).
7. Polyoxyethylene glycerol fatty acid ester represented by the following formula (VII):
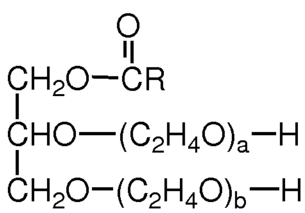

(VII)

[In the formula (VII), R represents an alkyl group having from 4 to 24 carbon atoms; a, b each means the added

molar number of ethylene oxide, and a + b indicates a number of from 2 to 150.]
Concretely, exemplary ones include POE glyceryl cocoate (as commercial products, "Unigly MK-207G") (by NOF Corp.).
8. Polyoxyethylene glyceryl isostearate represented by the following formula (VIII):
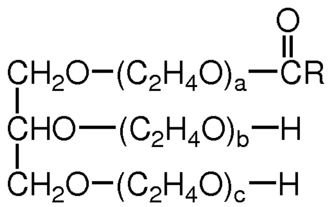

(VIII)

[In the formula (VIII), R represents an alkyl group having from 4 to 24 carbon atoms; a, b and c each means the added molar number of ethylene oxide, and a + b + c indicates a number of from 3 to 100.]
Concretely, exemplary ones include POE glyceryl isostearate (as commercial products, "Uniox GM-5IS") (by NOF Corp.).
9. Polyoxyethylene glyceryl triisostearate represented by the following formula (IX):
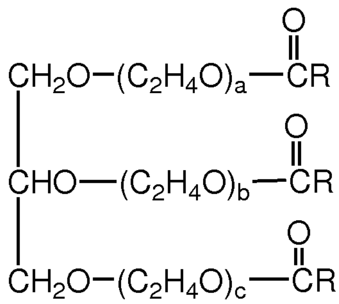

(IX)

[In the formula (IX), R each independently represents an alkyl group having from 4 to 24 carbon atoms; a, b and c each means the added molar number of ethylene oxide, and a + b + c indicates a number of from 3 to 100.]
Concretely, exemplary ones include POE glyceryl triisostearate (as commercial products, "Uniox GT-20IS") (by NOF Corp.).
10. Polyethylene oxide hydrogenated castor oil represented by the following formula (X):
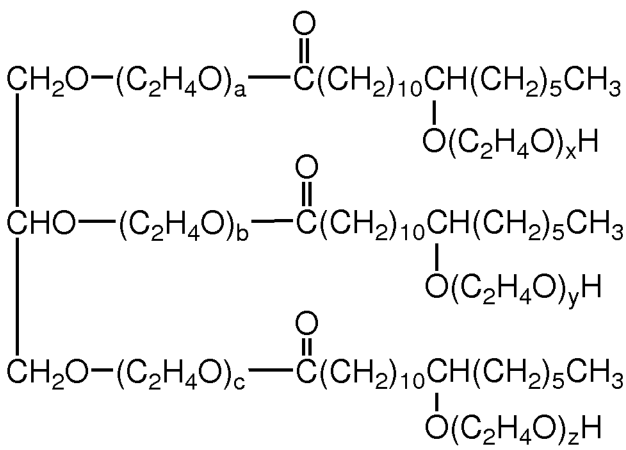

(X)

[In the formula (X), a, b, c, x, y and z each means the added molar number of ethylene oxide, and a + b + c + x + y + z indicates a number of from 2 to 150.]
Concretely, exemplary ones include POE hydrogenated castor oil 60 (as commercial products, "Uniox HC-60") (by NOF Corp.).

11. Polyoxyethylene alkyl ether represented by the following formula (XI):

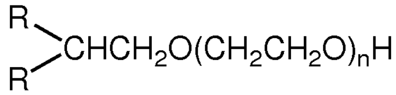

(XI)

[In the formula (XI), R each independently represents an alkyl group having from 4 to 24 carbon atoms; n means the added molar number of ethylene oxide, indicating a number of from 2 to 100.]
Concretely, exemplary ones include POE(20) octyl dodecyl ether (as commercial products, "Emalex OD-20") (by Nihon Emulsion Co., Ltd.), POE(25) octyl dodecyl ether (as commercial products, "Emalex OD-25") (by Nihon Emulsion Co., Ltd.).

Using a surfactant that is solid (solid form) at room temperature (25°C) effectively exerts hair styling property and hair set retention property. In addition, hair-washing-off capability could also be enhanced.

Regarding the standard indicating the solid (solid form) state of the above described surfactant, the hardness of the surfactant is preferably at least 20 in the invention, more preferably at least 40. The "hardness" as referred to herein is calculated as follows: First a melted sample is poured into a cylindrical glass bottle having a diameter of 3 cm and a depth of 3 cm (to a depth of at least 1 cm) and naturalized therein at 25°C for at least 12 hours. Next, using a curd meter (by Asukakiki) and at a sample temperature of 25°C, a load of 400 g is applied to the sample, and at the time when the pressure-sensitive shaft (diameter 1 mm) has stepped into the inside of 5 mm from the flat surface of the sample, the numerical value of the scale is taken as the hardness. A larger numerical value means a higher hardness.

The surfactant that is solid (solid form) at room temperature is preferably such that the weighted average HLB of all the nonionic surfactants is at least 10, more preferably at least 12, from the viewpoint of the solubility thereof in a water-base solvent such as water or alcoholic solvents and from the hair restyling property. HLB is calculated according to the Kawakami equation represented by the following numerical equation 1:

[Numerical Equation 1]

$$HLB = 7 + 11.7 \cdot \log(MW/MO)$$

(wherein MW means the molecular weight of the hydrophilic group moiety; and MO means the molecular weight of the oleophilic group moiety).

In the case where the surfactant that is solid at room temperature is incorporated, its amount to be used in the hair styling cosmetic composition of the invention is preferably from 1.5 to 12% by mass, more preferably from 2 to 10% by mass, most preferably from 2.5 to 10% by mass. When the amount is less than 1.5% by mass, then the surfactant could not sufficiently exert its property; but on the other hand, even though the amount is more than 12% by mass, not only there is no expecting a further improvement in efficacy that is commensurate with the increased amount of its use, but also the viscosity of the composition may increase, which is therefore unfavorable in point of the stickiness, and heavy finish of the hair.

The viscosity of the system of the hair styling cosmetic composition of the invention is at most 10, 000 mPa·s (at 25°C with B-type viscometer), preferably at most 1,000 mPa·s. In particular, in the case where the hair styling cosmetic composition of the invention is used by spraying it in the form of a mist or the like in use thereof, the viscosity is preferably at most 100 mPa·s. Not specifically defined, the lowermost limit of the viscosity is preferably at least 8 mPa·s or so from the viewpoint of the feeling in use.

The hair styling cosmetic composition of the invention contains (d) an aqueous solvent as the constituent ingredient. The aqueous solvent includes water, alcoholic solvents such as ethanol, etc., or their mixed solvents. The hair styling cosmetic composition of the invention is an aqueous one having a low viscosity, and its viscosity may be controlled, for example, by controlling the degree of polymerization of the copolymer to be incorporated therein, or by increasing or decreasing the amount of the polymers to be added, or by controlling the amount of the aqueous solvent to be incorporated therein.

Heretofore, water-based low-viscosity hair styling cosmetic compositions could hardly exhibit sufficient hair styling performance and hair restyling performance. Though having a low viscosity, the hair styling cosmetic composition of the present invention has secured sufficient both hair styling performance and hair restyling performance.

In addition to the above-mentioned ingredients, any other ingredient generally used in cosmetics, drugs or the

like may be optionally incorporated in the hair styling cosmetic composition of the invention within a range not detracting from the advantageous effects of the invention. The ingredient includes powder, higher fatty acid, UV absorbent, polyhydric alcohol, metal ion sequestrant, amino acid, organic amine, polymer emulsion, pH controlling agent, skin nutrient, vitamin, antioxidant, antioxidation promoter, and fragrance. These ingredients may be optionally suitably incorporated, and the hair styling cosmetic composition of the invention can be produced according to the intended preparation form thereof in an ordinary manner.

The hair styling cosmetic composition of the invention may be in any form of a water system or a dissolution system. Preferred use embodiments of the hair styling cosmetic composition of the invention include an aerosol-type hair spray, a non-aerosol-type hair spray, a hair mist, a hair mousse, a set lotion, a hair-styling gel, and a hair liquid.

As being water-based and having a low viscosity, the hair styling cosmetic composition of the invention exhibits excellent hair styling performance and hair restyling performance and therefore, even though in the form of a hair spray, a hair mist or the like that is used by spraying, the hair styling cosmetic composition can be widely and evenly applied onto hair, without clogging the spray nozzle of the spray container. An aerosol-type cosmetic product is generally charged in a spray container along with a propellant therein. As the propellant, herein usable is any and every propellant known in the field of aerosols, such as liquefied gas of propane, butane, pentane, dimethyl ether or the like, nitrogen, or compressed gas such as compressed air, etc. The amount of the propellant to be incorporated is preferably from 5 to 200% by mass relative to 100% by mass of the hair styling cosmetic composition (stock solution).

## EXAMPLES

The invention is described in detail with reference to the following Examples, but not limited thereto. Unless otherwise specifically indicated, the incorporated is expressed by % by mass (as actual content).

First described are the evaluation methods used in these Examples.

### [Viscosity]

A sample (100 to 200 mL) was put into a BL-type viscometer (rotor No. 2, number of revolution 60 rpm, 25 ± 2°C), and the sample viscosity was measured after the lapse of 1 minute from the start of the rotor rotation.

### [Hair styling property]

0.5 g of a sample was applied onto a bundle of black virgin hair (length 20 cm, mass 4 g), then rubbed into the hair with fingers, and thereafter evaluated according to the sensory test conducted by expert panelists (10 panelists) regarding the easiness in hair styling.

### [Hair restyling property]

0.5 g of a sample was applied onto a bundle of black virgin hair (length 20 cm, mass 4 g), then dried at room temperature for 1 hour, and evaluated according to the sensory test conducted by expert panelists (10 panelists) regarding the easiness in arranging the hair style by pinching, twisting and moving the hair.

### [Non-stickiness]

0.5 g of a sample was applied onto a bundle of black virgin hair (length 20 cm, mass 4 g), then rubbed into the hair with fingers, and evaluated according to the sensory test conducted by expert panelists (10 panelists) regarding the non-stickiness of the hair.

### [Smoothness]

0.5 g of a sample was applied onto a bundle of black virgin hair (length 20 cm, mass 4 g), then rubbed into the hair with fingers and finished styling, and thereafter evaluated according to the sensory test conducted by expert panelists (10 panelists) regarding the smoothness of the hair surface.

### [Light Finish of Hair]

0.5 g of a sample was applied onto a bundle of black virgin hair (length 20 cm, mass 4 g), then rubbed into the hair with fingers and finished styling, and thereafter evaluated according to the sensory test conducted by expert panelists (10 panelists) regarding the light finish of the hair.

<Rating Point>

5: Excellent
4: Good
3: Average (neither good or not good)
2: Somewhat not good
1: Not good

<Evaluation Standard>

Θ: At least 40 points as the total rating point.
O: From 30 points to less than 40 points as the total rating point.
Δ: From 20 points to less than 30 points as the total rating point.
×: Less than 20 points as the total rating point.

(Examples 1 to 15, Comparative Examples 1 to 6)

The samples shown in Tables 1 and 2 below were evaluated in point of the hair styling property, the hair restyling property, the non-stickiness, the smoothness, and the light finish of the hair according to the above-mentioned evaluation methods. The results are shown in Tables 1 and 2. In Tables 1 and 2, the following commercial products were used for the following ingredients.

- POP sorbitol (*1): "Uniol HS-1600D" (NOF Corp.).
- POE(10) methyl glucoside (*2) : "Glucam E-10" (by Lubrizol Japan Limited).
- Alkyl acrylate/diacetone acrylamide copolymer (*3) : "Plas Cize L-9909B" (by Goo Chemical Co., Ltd.).
- Vinylpyrrolidone/dimethylaminoethyl methacrylate copolymer diethyl sulfate salt (*4) : "H.C. Polymer 2" (by Osaka Organic Chemical Industry Ltd.).
- Vinylpyrrolidone/vinyl acetate copolymer (*5): "PVP/VA S-630" (by ISP).
- Silicone/polyether polyurethane resin (*6): "Yodosol PUD" (by AkzoNobel).
- POE(25) cetyl ether (*7): "Emalex 125" (by Nihon Emulsion Co., Ltd.).

[Table 1]

| Constitutive Ingredients | Examples | | | | | | | | | | |
|---|---|---|---|---|---|---|---|---|---|---|---|
| | 1 | 2 | 3 | 4 | 5 | 6 | 7 | 8 | 9 | 10 | 11 |
| Ion-Exchanged Water | to 100 | to 100 | to 100 | to 100 | to 100 | to 100 | to 100 | to 100 | to 100 | to 100 | to 100 |
| Ethanol | 60 | 60 | 60 | 60 | 60 | 60 | 60 | 60 | 60 | 60 | 60 |
| Sorbitol | 5 | - | - | - | 5 | 5 | 5 | 5 | 5 | 5 | 0.1 |
| Fructose | - | 5 | - | - | - | - | - | - | - | - | - |
| Polyethylene Glycol (molecular weight 1000) | - | - | 5 | - | - | - | - | - | - | - | - |
| Magnesium Chloride | - | - | - | 5 | - | - | - | - | - | - | - |
| POP sorbitol (*1) | - | - | - | - | 7 | - | - | - | - | - | - |
| POE(10) Methyl Glucoside (*2) | 7 | 7 | 7 | 7 | - | 7 | 7 | 7 | 7 | 7 | 12 |
| Alkyl acrylate/diacetone acrylamide copolymer (*3) | 2 | 2 | 2 | 2 | 2 | - | - | - | 2 | 2 | 4 |
| Vinylpyrrolidone/dimethylaminoethyl methacrylate copolymer diethyl sulfate salt (*4) | - | - | - | - | - | 2 | - | - | - | - | - |
| Vinylpyrrolidone/vinyl acetate copolymer (*5) | - | - | - | - | - | - | 2 | - | - | - | - |
| Silicone/polyether polyurethane resin (*6) | - | - | - | - | - | - | - | 2 | - | - | - |
| POE(25) Cetyl Ether (*7) (HLB=15, Hardness >100) | - | - | - | - | - | - | - | - | 3 | - | - |
| POE(60) Hydrogenated castor oil (HLB=14, Hardness10.95) | - | - | - | - | - | - | - | - | - | 3 | - |
| Viscosity (mPa·s/25°C) | 9 | 9 | 10 | 9.5 | 10 | 11 | 12 | 15 | 11.5 | 11 | 13.5 |
| Hair styling property (easiness in hair styling) | ○ | ○ | ◎ | ○ | ◎ | ○ | ○ | ○ | ◎ | ◎ | ○ |
| Hair restyling property (one hour after application) | ○ | ○ | ◎ | ○ | ○ | ○ | ○ | ○ | ◎ | ◎ | ○ |
| Non-stickiness | ○ | ○ | ○ | ○ | ○ | ○ | ○ | ○ | ○ | ○ | ○ |
| Smoothness | ◎ | ○ | ○ | ○ | ◎ | ◎ | ◎ | ◎ | ◎ | ◎ | ◎ |
| Light Finish of Hair | ◎ | ○ | ○ | ○ | ◎ | ◎ | ◎ | ◎ | ○ | ○ | Δ |

[Table 2]

| Constitutive Ingredients | Examples | | | | Comparative Examples | | | | | |
|---|---|---|---|---|---|---|---|---|---|---|
| | 12 | 13 | 14 | 15 | 1 | 2 | 3 | 4 | 5 | 6 |
| Ion-Exchanged Water | to 100 | to 100 | to 100 | to 100 | to 100 | to 100 | to 100 | to 100 | to 100 | to 100 |
| Ethanol | 60 | 60 | 60 | 60 | 60 | 60 | 60 | 60 | 60 | 60 |
| Sorbitol | 20 | 3 | 10 | 1 | - | 5 | 5 | 25 | 5 | 5 |
| Fructose | - | - | - | - | - | - | - | - | - | - |
| Polyethylene Glycol (molecular weight 1000) | - | - | - | - | - | - | - | - | - | - |
| Magnesium Chloride | - | - | - | - | - | - | - | - | - | - |
| POP sorbitol (*1) | - | - | - | - | - | - | - | - | - | - |
| POE(10) Methyl Glucoside (*2) | 3 | 25 | 12 | 3 | 7 | - | 7 | 7 | 30 | 7 |
| Alkyl acrylate/diacetone acrylamide copolymer (*3) | 2 | 1 | 0.1 | 12 | 2 | 2 | - | 2 | 2 | 15 |
| Vinylpyrrolidone/dimethylaminoethyl methacrylate copolymer diethyl sulfate salt (*4) | - | - | - | - | - | - | - | - | - | - |
| Vinylpyrrolidone/vinyl acetate copolymer (*5) | - | - | - | - | - | - | - | - | - | - |
| Silicone/polyether polyurethane resin (*6) | - | - | - | - | - | - | - | - | - | - |
| POE(25) Cetyl Ether (*7) (HLB=15, Hardness >100) | - | - | - | - | - | - | - | - | - | - |
| POE(60) Hydrogenated castor oil (HLB=14, Hardness10.95) | - | - | - | - | - | - | - | - | - | - |
| Viscosity (mPa·s/25°C) | 9.5 | 18.5 | 8 | 52 | 8 | 7 | 6.5 | 16.5 | 13 | 73.5 |
| Hair styling property (easiness in hair styling) | ○ | ○ | ○ | ◎ | △ | △ | × | ◎ | △ | ◎ |
| Hair restyling property (one hour after application) | ◎ | ◎ | ◎ | ○ | △ | × | ○ | ○ | ○ | × |
| Non-stickiness | ○ | △ | ○ | △ | ○ | △ | ○ | × | △ | × |
| Smoothness | ○ | ◎ | ○ | ○ | △ | △ | ○ | × | ○ | × |
| Light Finish of Hair | △ | ○ | △ | ◎ | ○ | ○ | × | × | × | × |

As obvious from the results in Tables 1 and 2, the hair styling cosmetic composition of the invention can exhibit both the effects of hair styling performance and hair restyling performance excellently and with a good balance therebetween, though having a low viscosity, and additionally can have the effects of non-stickiness, smoothness and light finish of the hair. On the other hand, the hair styling cosmetic composition beyond the scope of the invention could not exhibit all the advantageous effects of the invention.

Furthermore, the samples shown in Examples 16 - 19 were evaluated in point of the hair styling property, the hair restyling property, the non-stickiness, the smoothness, and the light finish of the hair as the same way according to the above-mentioned evaluation methods. The results are shown in Table 3.

(Example 16: Mist-type Hair Styling Agent)

| (Constitutive Ingredients) | (% by mass) |
|---|---|
| (1) Sorbitol | 10 |
| (2) POE(10) methyl glucoside (Glucam E-10, by Lubrizol Japan) | 5 |
| (3) Silicone/polyether-type polyurethane resin (Yodosol PUD, by AkzoNobel) | 2 |
| (4) L-menthol | 0.1 |
| (5) Citric acid (edible) | 0.1 |
| (6) POE(24)/POP(13) decyl tetradecyl ether (Unilube 50MT-2200B, by NOF) | 0.5 |
| (7) Fragrance | 0.1 |
| (8) Ethyl 4-hydroxybenzoate | 0.2 |
| (9) Ethanol | 50 |
| (10) Ion-exchanged water | to 100 |

Production Method:

(1), (2) and (5) were added in that order to (10) to prepare an aqueous part. (4), (6), (7) and (8) were added in that order to (9) to prepare an alcohol part. Subsequently, the alcohol part and (3) were added in that order to the aqueous part, and mixed them with stirring to prepare a mist-type hair styling agent.

(Example 17: Mist-type Hair Styling Agent)

| (Constitutive Ingredients) | (% by mass) |
|---|---|
| (1) Polyethylene glycol 4000 | 5 |
| (2) POP sorbitol (Uniol HS-1600D, by NOF) | 5 |
| (3) Vinylpyrrolidone/dimethylaminoethyl methacrylate copolymer diethyl sulfate salt (H.C. Polymer 2, by Osaka Organic Chemical Industry) | 3 |
| (4) POE(60) hydrogenated castor oil | 3 |
| (5) Diglycerine | 0.5 |
| (6) Wine extract | 0.1 |
| (7) Fragrance | 0.1 |
| (8) Ethyl 4-hydroxybenzoate | 0.2 |
| (9) Ethanol | 5 |
| (10) Ion-exchanged water | to 100 |

Production Method:

(2), (5), (3) and melted (1) were added in that order to (10) to prepare an aqueous part. (4), (6), (7) and (8) were added in that order to (9) to prepare an alcohol part. Subsequently, the aqueous part and the alcohol part were mixed with stirring to prepare a mist-type hair styling agent.

(Example 18: Mist-type Hair Styling Agent)

| (Constitutive Ingredients) | (% by mass) |
|---|---|
| (1) Maltitol | 7 |
| (2) POE(10) methyl glucoside (Glucam E-10, by Lubrizol Japan) | 10 |
| (3) Alkyl acrylate/diacetone acrylamide copolymer (Plas Cize L-9948B" (by Goo Chemical) | 3 |
| (4) POE(20) cetyl ether (Emalex 120, by NOF) | 5 |
| (5) Amino acid | 0.05 |
| (6) Fragrance | 0.1 |
| (7) Phenoxyethanol | 0.5 |
| (8) Ethanol | 5 |
| (9) Ion-exchanged water | to 100 |

Production Method:

(1), (2) and (5) were added in that order to (9) to prepare an aqueous part. Melted (4), (6) and (7) were added in that order to (8) to prepare an alcohol part. Subsequently, the aqueous part, the alcohol part and (3) were mixed with stirring to prepare a mist-type hair styling agent.

(Example 19: Mist-type Aerosol Hair Styling Agent)

| (Constitutive Ingredients) | (% by mass) |
|---|---|
| (1) Fructose | 5 |
| (2) Magnesium chloride | 5 |
| (3) POP sorbitol (Uniol HS-1600D, by NOF) | 2 |
| (4) POE(10) methyl glucoside (Glucam E-10, by Lubrizol Japan) | 2 |
| (5) Vinylpyrrolidone/vinyl acetate copolymer (PVP/VA S-630, by ISP) | 1 |
| (6) POE(20) cetyl ether (Emalex 120, by NOF) | 1 |
| (7) POE(60) hydrogenated castor oil | 1 |
| (8) 1,3-Butylene glycol | 0.5 |
| (9) Hydrolyzed wheat protein | 0.1 |
| (10) Propellant (nitrogen gas) | 40 |
| (11) Citric acid (edible) | 0.1 |
| (12) Fragrance | 0.1 |
| (13) Phenoxyethanol | 0.5 |
| (14) Ethanol | 30 |
| (15) Ion-exchanged water | to 100 |

Production Method:

(1), (2), (4), (5), (8) and (11) were added in that order to (15) to prepare an aqueous part. (3), (6), (7), (12) and (13) were added in that order to (14) to prepare an alcohol part. The aqueous part, the alcohol part and (9) were stirred and mixed, and (10) was charged therein to prepare a mist-type aerosol hair styling agent (aerosol spray).

[Table 3]

| | Example 16 | Example 17 | Example 18 | Example 19 |
|---|---|---|---|---|
| Viscosity (mPa·s/25°C) | 15 | 12.5 | 19 | 12.5 |

(continued)

| | Example 16 | Example 17 | Example 18 | Example 19 |
|---|---|---|---|---|
| Hair styling property (easiness in hair styling) | ○ | ⊖ | ⊖ | ○ |
| Hair restyling property (one hour after application) | ○ | ⊖ | ○ | ○ |
| Non-stickiness | ○ | ○ | ○ | ○ |
| Smoothness | ○ | ○ | ⊖ | ○ |
| Light finish of hair | ○ | ○ | ○ | ⊖ |

INDUSTRIAL APPLICABILITY

The hair styling cosmetic composition of the invention is excellent in hair styling property and hair restyling property, even though having a low viscosity, and is excellent in non-stickiness, smoothness, and light finish of the hair.

## Claims

1. A hair styling cosmetic composition comprising:

    (a) one or more substances being solid at room temperature (25°C) selected from among

        ($a_1$) a sugar alcohol,
        ($a_2$) a sugar,
        ($a_3$) a polyalkylene glycol polymer, and
        ($a_4$) an inorganic salt,

    in an amount of from 0.1 to 20% by mass,
    (b) a sugar derivative and/or a sugar alcohol derivative being liquid at room temperature, in an amount of from 0.1 to 25% by mass,
    (c) one or more film-forming polymers selected from among acrylic and vinylic film-forming polymers (but excepting ampholytic polymers) and urethanic polymers, in an amount of from 0.1 to 12% by mass, and
    (d) an aqueous solvent,

    wherein the viscosity of the system is at most 10,000 mPa·s (at 25°C with B-type viscometer).

2. The hair styling cosmetic composition as claimed in claim 1, wherein component (b) is one or more selected from among a sugar alkylene oxide addition polymer and a sugar alcohol alkylene oxide addition polymer, which are liquid at room temperature.

3. The hair styling cosmetic composition as claimed in claim 1 or 2, wherein component ($a_1$) is one or more selected from among maltitol, sorbitol, ribitol, mannitol, arabitol, galactitol, xylitol, erythritol and inositol.

4. The hair styling cosmetic composition as claimed in claim 1, wherein component ($a_3$) is a polyethylene glycol having a mass-average molecular weight of from 1,000 to 20,000.

5. The hair styling cosmetic composition as claimed in claim 1, wherein component (d) is water and/or an alcoholic solvent.

6. The hair styling cosmetic composition as claimed in claim 5, wherein the alcoholic solvent in component (d) is ethanol.

7. The hair styling cosmetic composition as claimed in claim 1, wherein component (a) contains at least component ($a_1$) and/or component ($a_3$) .

8. The hair styling cosmetic composition as claimed in claim 1, which has a viscosity of at most 100 mPa·s (at 25°C with B-type viscometer) and is used in the form of a fine mist by spraying the composition.

**INTERNATIONAL SEARCH REPORT**

International application No.
PCT/JP2011/064519

A. CLASSIFICATION OF SUBJECT MATTER
*A61K8/60*(2006.01)i, *A61K8/20*(2006.01)i, *A61K8/34*(2006.01)i, *A61K8/81*(2006.01)i, *A61K8/86*(2006.01)i, *A61K8/87*(2006.01)i, *A61Q5/06*(2006.01)i

According to International Patent Classification (IPC) or to both national classification and IPC

B. FIELDS SEARCHED

Minimum documentation searched (classification system followed by classification symbols)
A61K8/00-99, A61Q5/06

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | JP 3-261713 A (Kao Corp.),<br>21 November 1991 (21.11.1991),<br>claims; example 2(products 3, 4); example 3 (product 6); example 4<br>(Family: none) | 1,2,4-8<br>1-8 |
| Y | JP 11-100312 A (Shiseido Co., Ltd.),<br>13 April 1999 (13.04.1999),<br>claims 1 to 3; paragraphs [0010] to [0012], [0023]; example 17<br>(Family: none) | 1-3,5-8 |
| Y | JP 2006-69903 A (Mandom Corp.),<br>16 March 2006 (16.03.2006),<br>claims 1, 3; examples 1 to 5<br>(Family: none) | 1-3,5,7 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 September, 2011 (01.09.11) | 13 September, 2011 (13.09.11) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2011/064519

C (Continuation). DOCUMENTS CONSIDERED TO BE RELEVANT

| Category* | Citation of document, with indication, where appropriate, of the relevant passages | Relevant to claim No. |
|---|---|---|
| Y | JP 5-310535 A (Lion Corp., Osaka Eyazoru Kogyo Kabushiki Kaisha), 22 November 1993 (22.11.1993), claim 1; paragraphs [0009] to [0011] (Family: none) | 3 |
| Y | JP 2010-59157 A (Shiseido Co., Ltd.), 18 March 2010 (18.03.2010), claim 2; paragraphs [0010], [0016], [0029] (Family: none) | 1-8 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004505902 A
- JP 2007217314 A
- JP 3261713 A
- JP 2002167317 A
- JP 2006213706 A